# EUROPEAN PATENT SPECIFICATION

(11) **EP 0 442 223 B1**
(45) Date of publication and mention of the grant of the patent: **15.02.1995**
(21) Application number: 90314377.4
(22) Date of filing: 28.12.1990
(51) Int. Cl.: A61F 13/50, A61F 13/15

(54) **Disposable absorbent product**
Saugfähiger Wegwerfartikel
Article absorbant jetable

(30) Priority: 12.01.1990 US 464490
(43) Date of publication of application: 21.08.1991
(73) Proprietor: CHICOPEE, New Brunswick New Jersey 08903 (US)
(72) Inventor: Yang, Ching-Yun Morris, Princeton Junction, New Jersey 08550 (US)
(74) Representative: Fisher, Adrian John

(56) References cited:
- EP-A- 0 070 163
- EP-A- 0 070 164
- EP-A- 0 263 720
- EP-A- 0 317 058
- FR-A- 2 532 337
- GB-A- 2 170 108
- GB-A- 2 189 705
- US-A- 3 592 194
- US-A- 4 226 237

## Description

### Background of the Invention

The present invention relates to a new and improved disposable absorbent product, such as a urinary pad, having high liquid impact capacity, high liquid retention, and which allows the skin of the wearer to remain dry.

Disposable absorbent products have been known for some time, including such products as disposable diapers, sanitary napkins, wound dressings, bandages, incontinent pads, and the like. These products incorporate an absorbent batt which is used to absorb and hold or contain body fluids. Initially in many of these products, especially diapers and sanitary napkins, the absorbent batt comprised what is termed "wadding" or plies of tissue. The wadding was disposed between a liquid-impermeable backing and a liquid-permeable facing and the plies of tissue were used to absorb and, hopefully, contain the liquid within the product. A diaper which utilizes such an absorbent batt is disclosed in U.S. Reissue Patent No. 26,151.

The wadding type of product was replaced, for the most part, by an improved absorbent batt which comprises what is termed "fluffed wood pulp fibers". This absorbent batt comprises a layer of individualized wood pulp fibers with the layer having substantial thickness. A diaper which incorporates such fluffed wood pulp absorbent batt is described in U.S. Patent No. 2,788,003. This diaper had improved absorbent capacity and somewhat better containment than a diaper using a wadding layer. Also, the fluffed wood pulp layer is quite soft, flexible, and conformable, and, hence, produces an improved diaper over diapers using wadding as the absorbent layer.

Although fluffed wood pulp absorbent batts have a good absorptive capacity, the efficiency with which the capacity is used in a diaper or sanitary napkin is poor. The reason for this is that the fluid to be absorbed is generally deposited in a localized area of the absorbent batt, and the ability of the fluid to move along the plane of the batt is poor. The fluid tends to follow a radial wicking path and consequently moves to the closest edge of the batt where it generally is no longer contained and the product leaks.

In designing a disposable urinary device, one must be mindful of the special problems of an incontinent adult. First, the void of an adult generally is much greater in volume than that of an infant, so a device with greater absorptive capacity than that of an infant's diaper is needed. Second, a bulge under clothing is accepted by society for an infant, but the ambulatory adult with an incontinence problem desires a product which is not apparent through ordinary clothing. Third, the proportions and shape of the legs and torso of the adult differ considerably from those of an infant. Therefore, a mere enlargement of an infant diaper is not a satisfactory product.

A number of years ago, "superabsorbent materials", i.e., materials which will absorb many times their weight of liquid, were developed. Since the development of such materials, attempts have been made to incorporate them in absorbent products such as diapers to enhance the absorption performance of these products. Theoretically, a minimum amount of superabsorbent incorporated in a product would make that product perform as well or better than the prior art products. Perhaps one of the first products to incorporate such a superabsorbent material is a disposable diaper as disclosed in U.S. Patent No. 3,670,731. This patent discloses an absorbent dressing comprising an absorbent layer sandwiched between a permeable facing and an impermeable backing sheet. The absorbent layer contains water-insoluble crosslinked hydrocolloid polymer as the superabsorbent material.

Absorbent products employing superabsorbent materials as mentioned above have not to date met with complete success. Although the presence of the superabsorbent materials should greatly increase the liquid holding capacity of the product, difficulties have been encountered in handling the materials and in making a product in which the superabsorbent is evenly dispersed. There thus remains a need for highly absorbent materials which do not pose these problems.

The use of peat moss, alone or in combination with other fibrous materials, in absorbent products has previously been suggested. U.S. Patent No. 4,170,515 to J.M. Lalancette discloses a method for bleaching peat moss, thereby making it suitable for use in absorbent dressings and the like. U.S. Patent No. 4,226,237 to Y. Levesque discloses a layered absorbent structure including a first layer comprising cellulose fibers and, adjacent to the first layer, a second layer comprising, in admixture, peat moss and finely ground mechanical wood pulp. U.S. Patents Nos. 4,215,692 and 4,507,122, both issued to Levesque, disclose that peat moss, in combination with mechanical wood pulp, can be formed into a low density board, dried, and then compressed to form a thin, flexible, absorbent board which may be used directly in absorbent products.

U.S. Patent No. 4,473,440 to Ovans discloses a peat moss containing board which is manufactured by first conditioning the board to a specific water content and then densifying the board by calendaring between rollers. U.S. 4,676,871 discloses an air laid peat moss board suitable for use in absorbent products which is made by harvesting and individualizing peat moss, drying the peat moss, entraining the peat moss in a gas stream, and then condensing the entrained peat moss to form a low density board.

Numerous designs for disposable urinary devices have been suggested. U.S. Patent No. 4,685,914 discloses a disposable urinary pad which utilizes superabsorbent material. The pad disclosed in this patent comprises a liquid-impermeable, substantially flexible shell containing a superstructure consisting essentially of a fibrous web of hydrophobic, wet resilient, dry resilient fibers and an absorbent medium in intimate contact with at least a portion of said superstructure and at least a portion of said shell. In a preferred embodiment, the superstructure is a corrugated fibrous web, e.g., of polyester fibers, and the absorbent medium is a superabsorbent material.

U.S. Patent No. 4,501,586 discloses an absorbent structure comprising a moisture-impermeable backing, an absorbent batt and a moisture-permeable cover covering at least the side opposite the moisture-impermeable backing. The absorbent batt is of loosely-compacted, cellulosic fibers and is provided with a reservoir having a capacity of at least 10cc. The reservoir is formed by compression of the fibers in the reservoir zone and is located so that the product, when worn, retains the proper shape.

U.S. Patent No. 4,731,070 discloses an absorbent article particularly suitable for use by male and female incontinents. The absorbent article includes a urine receptacle pocket offset to one end of the product and formed by folding the product and adhering together portions of a moisture impervious sheet that are folded over side marginal edges of an absorbent batt.

GB-A-2 170 108 discloses an absorbent product having a liquid-impermeable backing sheet, an absorbent unit, a liquid permeable facing adhered to said backing sheet to entrap the absorbent unit therebetween, and elastic disposed adjacent the edges of the product to form the product into a U-shape. The absorbent unit comprises two layers of different stiffness.

The present invention provides a new and improved disposable absorbent urinary device which possesses a large liquid storage capacity, which is conformable to the body of the wearer and comfortable in use, which is not readily apparent through normal clothing and which more efficiently utilizes the liquid retentive capacity of the absorbent materials.

### Summary of the Invention

The present invention is defined in Claim 1.

The device of this invention has a high impact capacity, i.e. it readily accepts a relatively large volume of liquid without leakage or liquid spill over. The device also has a high liquid-holding capacity, i.e. once liquid enters the absorbent unit, it is absorbed and retained therein. In addition, the device maintains its surface dry thereby keeping any moisture away from the skin of the wearer. Finally, the device is compact in size so that it is easy to carry and wear and is less apparent under the wearer's clothing.

### Brief Description of the Drawings

Figure 1 is a perspective view of one embodiment of a disposable urinary device of the present invention.

Figure 2 is a cross-sectional view taken along line 2-2 of Figure 1.

Figure 3 is a schematic side elevational view of a production line for producing the disposable devices of this invention.

Figure 4 is a top plan view of a series of devices during the production stage and prior to their separation from one another into individual devices.

Figure 5 is a cross-sectional view taken along line 5-5 of Figure 4.

Figure 6 is an exploded cross-sectional view showing the structural components of the device of Figure 1 prior to assembly.

### Detailed Description of the Invention

A key feature of the absorbent device of this invention is the provision in the absorbent unit of first and second portions of differing degrees of stiffness. The stiffer first portion of the absorbent unit serves as a skeleton for the absorbent product, functioning in combination with the elastic means to impart the aforementioned, flattened U-shaped cross-sectional configuration to the crotch portion of the device. The first portion, also sometimes referred to herein as the "absorbent member", must be sufficiently rigid so that when the elastic means in the side margins of the device are contracted, there is no distortion of the rigid first portion in the transverse direction, i.e., there is no significant change in the width of the first portion. With the contraction of the elastic means, the longitudinally extending side margins of the less rigid second portion are caused to extend at least partially up the side walls of the device in the crotch portion to provide cushioning for comfort and, in effect, to form a gasket to entrap free liquid, preventing it from leaking out of the device. This unique structure conforms to and closely seals to the body of the wearer to provide comfort, minimum distortion and reduced incidence of leakage.

The stiffer first portion of the absorbent unit preferably has a transverse stiffness (i.e., bending stiffness measured in the direction perpendicular to the longitudinal axis) of at least about 2000 mg but preferably not more than about 8000 mg, and the less rigid second portion preferably has a transverse stiffness not greater than about 3000 mg and preferably not less than 200 mg. In all cases, however, the transverse stiffness of the first portion must be greater than the transverse stiffness of the second portion. The stiffnesses mentioned herein are measured using a Gurley Stiffness tester and standard test procedure described in detail later in this specification. If the transverse stiffness of the rigid first portion is less than about 2000 mg, the flattened U-shaped configuration in the crotch region may be distorted, thus increasing the chances for leakage. If the transverse stiffness of the second, less rigid portion is greater than about 3000 mg, the second portion is less likely to provide the aforementioned gasketing effect, thus reducing conformability of the device to the body of the wearer and increasing the chances for leakage to occur.

The less rigid, second portion, also sometimes referred to herein as the "receiving layer", comprises a material which is capable of absorbing body fluid more rapidly than the absorbent member but which does not exhibit the overall absorptive capacity of the absorbent member. Nonetheless, the second portion has an absorptive capacity of at least 12 g water per gram. The receiving layer is positioned on top of the absorbent member and in use is disposed upwardly so as to face the body of the wearer of the absorbent product. The receiving layer functions to receive and hold voided body liquid, e.g. urine, until said liquid is absorbed by the underlying absorbent member. With the passage of time (the amount of which is a function of the rate at which the absorbent member can take up liquid), the absorbent member absorbs liquid from the receiving layer. The receiving layer, which in use is closer to the wearer's body than the absorbent member, becomes relatively drier. As a result, the wearer of the device is more comfortable and the receiving layer is able to receive, without leakage, a further discharge of liquid.

In the present invention, the first portion of the absorbent unit and the second portion of the absorbent unit exist as separate, individual, discrete layers of absorbent material. In a preferred embodiment, the stiffer first portion comprises a three-layered composite in which the center layer is a blend of 95% by weight peat fibers and 5% by weight of 1.3 cm (1/2"), 1.5 denier polyester fibers. The outer layers of the three-layer composite are wood pulp fiber layers, each of said layers comprising about 3.3% by weight of the total weight of the composite. The density of the three-layer composite is about 0.35 g/cm³. As indicated earlier, the three-layered composite can be prepared by the method disclosed in U.S. 4,676,871, or by other suitable techniques known in the art for this purpose. At this stage, the three-layer laminate is as a practical matter too stiff to be used in the present invention so it is "tenderized", or made less stiff, by the perfembossing technique disclosed in U.S. Patent No. 3,817,827. At the same time, it is necessary, as indicated earlier herein, that the first portion have a stiffness of at least 2000 mg so that the flattened U-shaped cross-section in the crotch region of the inventive device is attained. Accordingly, the perfembossing procedure is carried out in order to reduce stiffness, thereby to increase comfort in use, but not to such an extent that the transverse stiffness of the perfembossed laminate is reduced to a value of less than about 2000 mg.

It will be understood by those skilled in the art that other absorbent materials may be used to make the first portion of the absorbent unit. Such materials include, e.g. comminuted wood pulp fibers; blends of wood pulp fibers with other natural or synthetic fibers (such as, respectively, cotton fibers or polyester fibers) and/or the superabsorbent materials well-known to those skilled in the art. Whatever the materials used, the first portion must have a transverse stiffness of at least about 2000 mg and have a sufficiently high absorptive capacity to absorb the rather large volumes of liquid frequently excreted by an incontinent adult.

In the preferred embodiment, the less rigid second portion, i.e. the receiving layer, comprises a blend of 80% by weight of comminuted wood pulp fibers and 20% by weight of a bicomponent fiber. The bicomponent fiber, which has a polyester core and a polyethylene sheath, has a denier of 3.0 and is about 3.2 cm (1¼ inches) long. This fiber is commercially available in the U.S. from American Enka. The receiving layer was made as follows. The bicomponent fibers were carded into a web having a basis weight of 68 g/m² (2 oz/yd²). The resulting bicomponent fiber web and a wood pulp board were fed into the dual rotor apparatus disclosed in U.S. Patents 3,768,118 and 3,740,797, to form a blended fibrous web having a basis weight of about 271 g/m² (8.0 oz/yd²). This blended fibrous web was then passed at a rate of 9.1 m/min (10 yd/min) through a forced air oven operating at a temperature of 149°C (300°F) wherein the sheath portion of the bicomponent fibers was caused to fuse to thereby stabilize the constituent fibrous components and provide the receiving layer. This receiving layer had an absorptive capacity of 16 grams of water for each gram of receiving layer and a transverse stiffness of about 400 mg. The thickness of the layer was about 6mm.

It will be appreciated that various absorbent materials or blends thereof may be used in place of those employed in the above-described receiving layer. For example, the weight ratio of wood pulp fibers to bicomponent fibers may vary from 50:50 to 95:5. Other thermoplastic binder fibers may be used in place of the aforementioned bicomponent fibers. The receiving layer must have a liquid absorbent capacity sufficient to quickly take up that volume of urine - frequently as much as 120 cc - which may be discharged by an adult incontinent. In addition, the receiving layer must have some deformability in its thickness so as to be able to perform its above-mentioned gasketing function.

The absorbent member is generally cut in a rectangular shape, with or without rounded corners, and may comprise one or more layers of absorbent material or a single layer of such material folded over on itself to provide two or more layers. In any event, the width of the first portion should be substantially equal to the desired width of the absorbent pad in the crotch portion. The less rigid receiving layer is also generally rectangular in shape, with or without rounded corners, and is wider than the absorbent member. The receiving layer is wide enough, when placed on top of the absorbent member, to extend beyond the lengthwise edges of the absorbent member and at least partially up the side walls of the pad in a manner to be explained in more detail hereinafter.

Referring now to the drawings, there is shown the preferred embodiment of a disposable absorbent urinary device in accordance with the teachings of the present invention. Device 10 comprises a liquid-impermeable backing sheet 12, an absorbent unit 13, and a liquid-permeable facing sheet 14. The absorbent unit, to be described in more detail hereinafter, is sandwiched between backing sheet 12 and facing sheet 14. The outer surface 12b of backing sheet 12 is provided with a plurality, and specifically three, spaced-apart strips of adhesive 16 which are protected, prior to use of the device, by a readily removable cover sheet 19. Adhesive strips 16 are used to secure device 10 to an undergarment of a wearer during use.

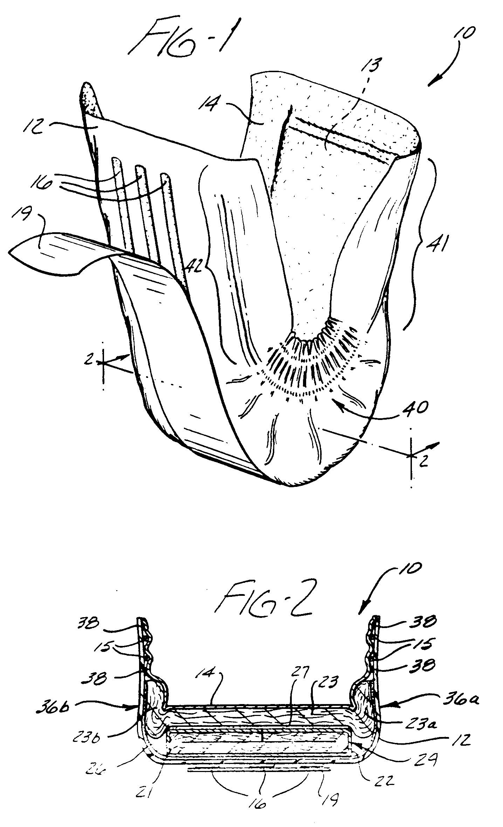

As seen in Fig. 1, device 10 has a crotch portion 40, an adjoining front portion 41 and an adjoining back portion 42. The device further comprises elastic means in the form of elastic monofilaments 15 inserted and secured between backing sheet 12 and facing sheet 14 at each side of device 10 in its crotch portion 40. These elastic monofilaments are secured, as with a hot melt adhesive, in a stretched condition during manufacture. In Fig. 1, the elastic monofilaments have been allowed to assume their relaxed, elastically contracted state. The contracted elastics, cooperating with the rigid first portion of the absorbent unit, provide device 10 with the end-to-end U-shaped configuration shown in perspective in Fig. 1.

In the preferred embodiment under discussion, liquid-impermeable backing sheet 12 comprises a film of polyethylene having a thickness of about 0.036 mm (1.4 mils). Other liquid-impermeable materials may be used in place of polyethylene. Liquid-pervious facing sheet 14 is a nonwoven fabric, preferably one comprising hydrophobic fibers such as 3 denier, 3.8 cm (1½ inch) polypropylene fibers, and having a basis weight of about 24 g/m² (0.7 oz/yd²). The fibers comprising the nonwoven fabric may be held in place by the use of a polymeric binder, hydroentanglement or any other suitable bonding method well known to those skilled in the art.

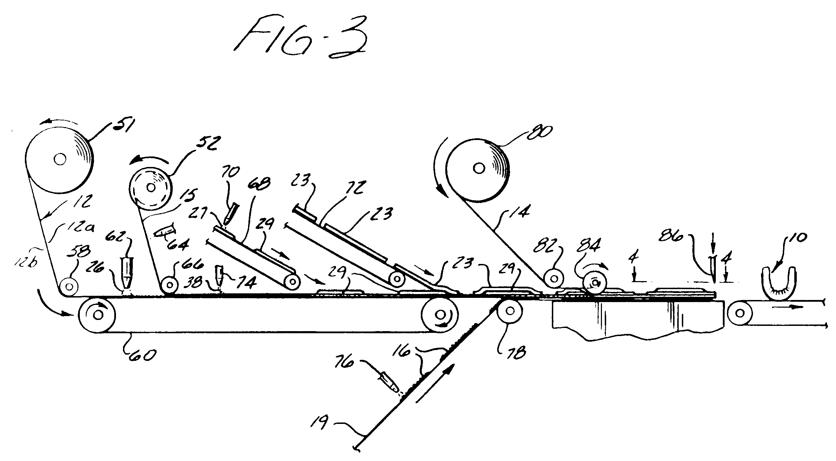

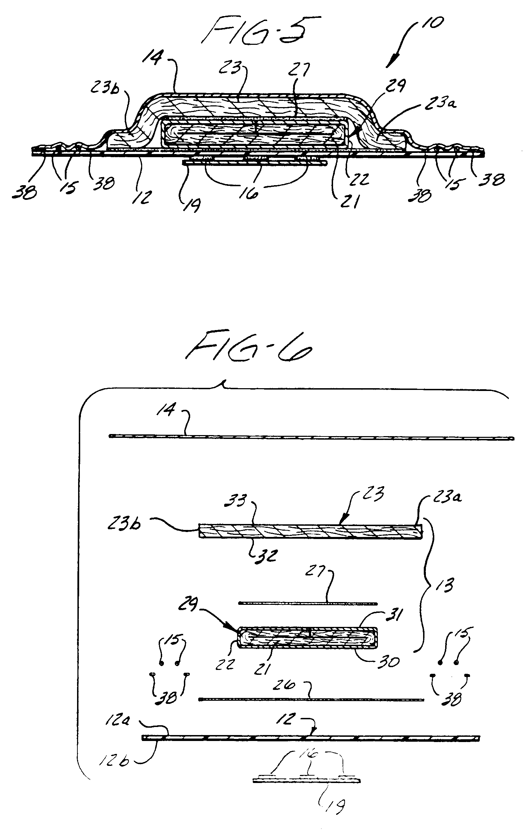

As best seen in Figs. 2 and 6, absorbent unit 13 of device 10 comprises a rigid first portion 29 of liquid absorbent material, also called the absorbent member. One function of first portion 29, as indicated earlier herein, is to define the width of device 10 in its crotch portion and, in order to achieve this, the rigid first portion of absorbent material has a transverse stiffness of at least about 2000 mg. A second function of first portion 29 is to ultimately absorb from the receiving layer (to be described hereinafter) the liquid which is discharged into device 10 by the wearer. Thus, first portion 29 must have a liquid absorption capacity sufficiently high to absorb the relatively large amounts of urine which can be expected to be discharged, either by a single large void or by two or more smaller voids, by an incontinent adult over the period of time the urinary device would be worn.

In addition to the aforementioned functions of defining the width of the device and of absorbing liquid discharged into the device, first portion 29 must also function to retain and immobilize said discharged liquid under the pressure encountered as a result of the normal activities of the wearer of the device. When the first portion functions to retain and immobilize liquid, the possibility of discharged liquid flowing back into and through the receiving layer and facing sheet and rewetting the body of the wearer is greatly reduced.

In the preferred embodiment, first portion 29 comprises the aforementioned three-layered composite, i.e. a composite in which the center layer is a blend of 95% by weight peat fibers and 5% by weight of polyester fibers and in which the outer layers are wood pulp fibers. This composite material, which is identified by numeral 21 in the accompanying drawings, has a thickness of 1.5 mm, a transverse stiffness of 5000 mg, a density of 0.35 g/cm³, and can ultimately absorb and retain about 12-13 times its own weight of water. As seen in Figs. 2 and 6, three-layered composite 21 is folded in a "C-configuration" and wrapped in a layer of tissue 22 having a basis weight of about 20 g/m² (0.6 oz/yd²). The aforementioned "C-configuration" is easily obtained by folding the sides of the composite material over its center portion until those sides abut each other in the manner shown in the drawings. It will be understood that the stiffness required for rigid first portion 29 is provided by three-layered composite 21. Tissue wrap 22 is a preferred although not a necessary structural element of first portion 29. While tissue 22 makes no significant contribution to stiffness, it does serve to contain fiber fragments originating from the preferred composite material 21.

Absorbent unit 13 further comprises a second portion 23 of liquid absorbent material, also called the receiving layer. The stiffness of second portion 23, i.e. the receiving layer, must be less than the stiffness of rigid first portion 21, i.e. the absorbent member. One requirement of the receiving layer, as its name suggests, is that it must be able to receive and temporarily hold a relatively large volume of discharged urine until said urine is ultimately absorbed by the absorbent member. Another requirement of the receiving layer is that it be less stiff, and preferably considerably less stiff, than the absorbent member. The substantially reduced stiffness of the receiving layer allows it, under the forces applied by the retracted elastic elements 15, to assume the configuration shown in Fig. 2. There it will be seen that at least part of receiving layer 23 extends up the sidewalls 36a, 36b comprising crotch portion 40 of disposable urinary device 10. This extension of at least part of the receiving layer upwardly along the sidewalls of device 10 provides a gasketing means which prevents discharged urine from leaking from the device. In addition, and as also seen in Fig. 2, a part of receiving layer 23 extends partially over the side edges of absorbent member 29 to provide cushioning at the sides of device 10 when it is in use.

In addition, the upwardly extending portions of the receiving layer, owing to their reduced stiffness, readily allow device 10 to conform to the wearer's body and render it more comfortable during use. Further, the width of receiving layer 23 is greater than the width of absorbent medium 21 so that, as can be seen in the drawings, its longitudinal side portions 23a, 23b extend beyond the sides of the absorbent member 29. The length of receiving layer 23 should be at least equal to that of absorbent member 29. In the preferred embodiment, receiving layer 23 is somewhat longer than absorbent member 29.

In the preferred embodiment, the less rigid second portion or receiving layer 23 comprises the aforementioned blend of 80% by weight of comminuted wood pulp fibers and 20% by weight of bicomponent fibers.

Still referring especially to Figs. 2 and 6, it will be seen that the lower surface 30 of tissue-wrapped, C-folded absorbent member 29 is adhered to the inner surface 12a of backing sheet 12 by a layer of adhesive 26. This adhesive layer extends transversely beyond the longitudinally extending side edges of absorbent member 29 so as to also secure the lower surfaces 32 of the longitudinally extending side marginal portions 23a, 23b of receiving layer 23 to the inner surface of the backing sheet. Lower surface 32 of receiving layer 23 is secured to upper surface 31 of the tissue-wrapped, C-folded absorbent member 29 by a layer of adhesive 27. Liquid pervious facing sheet 14 overlies and is in contact with upper surface 33 of receiving layer 23. Facing sheet 14 and receiving layer 23 are held together in the mutual area of contact by the frictional engagement of their fibers. A small amount of adhesive maybe applied between facing sheet 14 and receiving layer 23, if such is desired to prevent slipping of the facing sheet with respect to the receiving layer.

As indicated earlier, elastic monofilaments 15, two on either side of device 10, are secured in a stretched condition between facing sheet 14 and backing sheet 12 in the crotch portion of the device. These elastic elements are held in place by small amounts of adhesive (not illustrated in the drawings) applied intermittently along the length of the monofilaments, said intermittently applied adhesive also serving to help hold the facing sheet to the backing sheet in crotch region 40. The facing sheet and backing sheet are primarily held together at the sides of device 10 by longitudinally extending lines of adhesive 38. There are two such lines of adhesive 38 on each side of the device and they extend throughout the length of device 10. At each side of the device, there is one line of adhesive 38 on either side of two monofilaments 15, 15.

It can be seen in Fig. 2 that rigid first portion (absorbent member) 29 of absorbent unit 13 supports the flattened U-shaped configuration in the crotch region of device 10. Part of less rigid second portion (the receiving layer) 23 extends upwardly along the side walls 36a, 36b of the device to provide a gasket to seal the device to the wearer's body and prevent leakage. Generally, the height of sidewalls 36a, 36b in the crotch portion of the urinary device shown in Fig. 2 is at least about 1 inch (2.54 cm). The angle at which the side walls extend from the bottom of device 10 is illustrated in Fig. 2 as about 90° but as a matter of practice this angle may vary to some extent. As mentioned, part of less rigid second portion 23 extends partially over the side edges of absorbent member 29 as seen in Fig. 2.

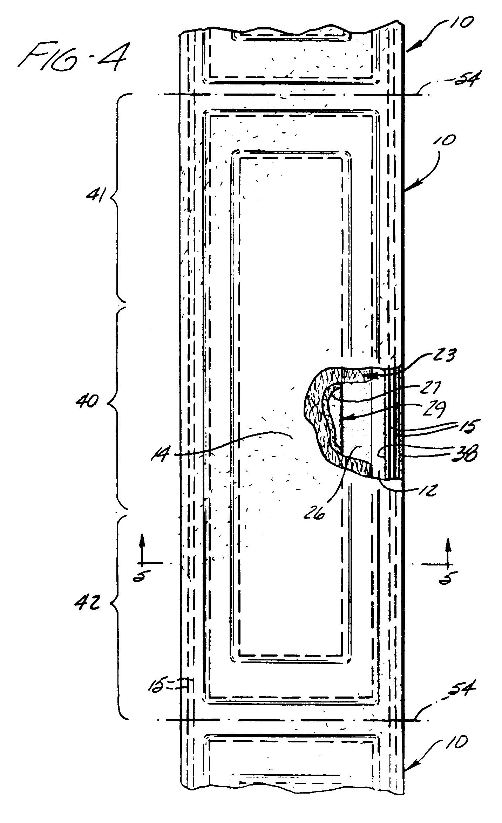

Fig. 3 is a schematic diagram showing a production line for preparing the disposable absorbent urinary device 10 illustrated in Figs. 1 and 2. Liquid-impermeable polyethylene backing film 12 approximately 6.25 inches (15.9 cm) in width is let off from supply roll 51 and passed under idler roller 58 onto the upper surface of an endless conveyor belt 60. Film 12 is coated with hot melt adhesive 26 at adhesive application station 62, said adhesive being applied at a coverage of about 6.8 g/m² (0.2 oz/yd²). A pair of elastic elements 15,15 is supplied to each side margin of backing film 12 from supply rolls 52. After leaving supply roll 52, each elastic monofilament is coated with an adhesive at selected intervals along its length at adhesive application station 64. It will be understood that the adhesively coated portions of the extended elastic elements 15 are caused to register with those portions of the backing sheet which will thereafter correspond to the crotch portions 40 of individual devices 10. The adhesive coated monofilaments are led under idler roller 66 where they are pressed into adhering contact with upper surface 12a of the backing film. The elastic monofilaments are stretched about 100% and secured to the backing film in their elastically extended state. The two monofilaments at each side are spaced about 6.4 mm (0.25 inch) apart. The distance between the innermost monofilament on one side of the film and the innermost monofilament on the opposite side of the film is about 4½ inches (about 10.2 cm). Individual absorbent members 29 are supplied at suitable intervals to the upper surface of backing film 12 by first feeder 68. A layer of adhesive 27 is applied to each individual absorbent member 29 at adhesive application station 70 prior to its being deposited on the backing film. Individual receiving layers 23 are supplied from second feeder 72 and are deposited in position atop individual absorbent members 29. Adhesive 27, previously applied to the upper surface of absorbent members 29, serves to adhere each respective receiving layer 23 to its corresponding underlying absorbent member 29. Previously applied adhesive 26 serves to secure lower surface 30 of absorbent member 29 to backing film 12 and also serves to secure side portions 23a,23b to backing film 12. Two continuous lines of adhesive 38 are applied to upper surface 12a of the backing film at each side thereof.

These lines of adhesive are applied at adhesive application station 74. As shown in Fig. 6, the two elastic monofilaments 15,15 at each side of film 12 are disposed between a pair of adhesive lines 38,38.

Protective covering 19, supplied from a let-off roll (not illustrated on the drawings), is coated with three spaced lines of adhesive 16 at coating station 76 and the adhesive coated protective covering is then pressed against the lower surface 12b of backing film 12 by passing it over idler roller 78. Liquid permeable facing 14 is supplied from supply roll 80, passed under idler roller 82 to be positioned on top of the previously assembled components. The assemblage is passed under a compression roller 84 to enhance adhesive bonding of the various components. The continuous strip of devices 10 is then severed into individual disposable absorbent urinary devices 10 by cut-off knife 86. After being severed, the individual urinary devices 10, under the influence of contracted elastic monofilaments 15 in cooperation with rigid absorbent member 29, assume the U-shaped end-to-end configuration illustrated in Fig. 1.

Fig. 4 is a top plan view of three joined devices 10, produced as just described with reference to Fig. 3, prior to being severed into individual urinary devices 10. Fig. 4 shows the relative widths and lengths of absorbent member 29 and receiving layer 23 as well as the location of elastic monofilaments 15 and continuous adhesive lines 38 along the longitudinal sides of device 10. It will be understood that each elastic monofilament 15 is intermittently secured between backing sheet 12 and facing sheet 14 only in crotch portion 40 of device 10. After the series of joined devices is severed into individual devices 10 along lines of severance 54, the elastic monofilaments return to a contracted state, thereby forming gathers in crotch portion 40. At the same time, the contracted elastic monofilaments cooperate with relatively rigid absorbent member 29 to give individual device 10 the U-shaped end-to-end configuration seen in Fig. 1 and to provide crotch region 40 with the generally flattened U-shaped cross-sectional configuration seen in Fig. 2.

In the embodiment shown in Figs. 1-6, the elastic monofilaments 15 were obtained from North American Globe Company, Fall River, Massachusetts. Each elastic monofilament was substantially circular and had a cross-sectional area of 0.0004"² (0.26 mm²). When elastic monofilament 15 was extended to 200% of its original length, it was found to exert a retractive force of about 75 grams. This retractive force was equal to that force required to stretch elastic element 15 to 200% of its unstretched length. The force required to stretch the elastic monofilament to 200% of its unstretched length was measured on an Instron Tester. The elastic monofilament was clamped between the two jaws of an Instron Model 1122 tester, with the two jaws being separated by a distance of 3 inches (7.62 cm.). The force required to then stretch the monofilament from a length of (3.0 inches) to a length of 15.2 cm (6.0 inches) (i.e. to stretch the monofilament to 200% of its original unstretched length) was taken as the retractive force.

It will be recognized by those skilled in the art that the amount of retractive force supplied by the elastic means used in the sides of device 10 will vary depending on the stiffness of the absorbent member 29 in its lengthwise direction. As the stiffness of the absorbent member 29 in its lengthwise direction increases, the retractive force required to be provided by the elastic means will also increase. It will be evident that the important thing is that the rigidity of absorbent member 29 and the retractive force exerted by the elastic means used at the sides of the device be selected so as to provide both the U-shaped end-to-end configuration shown in Fig. 1 and the generally flattened U-shaped cross-sectional configuration for crotch portion 40 shown in Fig. 2.

The elastic means in each side margin of the device must be sufficiently extended prior to their securement between the backing sheet and the facing sheet so that, when the elastic means retract at the time of cutting into individual devices, they function to bring front portion 41 and back portion 42 toward each other around the transverse center-line of device 10. Stated differently, it will be recognized that the elastic means in the side margins of the device must exert a sufficiently high retractive force and be sufficiently extended at the time of their securement between the backing sheet and the facing sheet so that, when they later retract, they function to impart to individual device 10 the end-to-end U-shaped configuration shown in perspective in Fig. 1 and in side view at the far right-hand side to Fig. 3.

In the preferred embodiment discussed herein, each elastic monofilament 15 had a substantially circular cross-section, a diameter of 0.023 inch (0.6 mm), a retractive force of 75 grams and was extended to 200% of its original length before its securement between the backing sheet and the facing sheet in the crotch portion of the device. Those skilled in the art will be able to select other elastic means which, although this cross-sectional configuration and area, retractive force and % extension may be different from those described above for monofilament 15, will nevertheless perform the above-mentioned functions.

Fig. 5 is a cross-sectional view of device 10 as shown in Fig. 4, prior to severing and contraction of the elastic monofilaments 15. Fig. 5 shows the three-layer composite 21 folded on itself in a C-configuration and wrapped in tissue 22. Fig. 5 also shows how the receiving layer 23 extends over the side edges of absorbent member 29 and is adhered to the liquid impermeable backing sheet 12. Adhesive means 38 are placed at the side edges of the device, between the liquid impermeable backing sheet 12 and the liquid permeable facing sheet 14.

Fig. 6 is an exploded cross-sectional view showing the relative positions and sizes of the elements of a device of this invention prior to assembly. Three-layer composite 21, wrapped in tissue 22, is narrower than receiving layer 23 so that said receiving layer extends beyond the side edges of absorbent member 29 and is adhered to backing 12. Adhesive means 38 are placed beyond the side edges of receiving layer 23 and between the backing 12 and liquid permeable facing 14. Layers of adhesive 26 and 27 serve to join the respective members together, and adhesive strips 16 serve to adhere the device to the undergarment of the wearer.

Turning now to a more detailed description of the components of the device of this invention, the sheet of tenderized peat moss which can be utilized as the absorbent member may be made by methods well known in the art, such as those disclosed in the previously mentioned patents. Generally, the raw peat moss material utilized is peat moss of the sphagnum variety and is preferably capable of absorbing at least about 15 times, preferably about 20 times, its weight in water. The peat moss is generally screened and then separated into a usable fraction and peat fines. The screened peat moss may be combined with other absorbent materials, preferably fibrous and cellulosic in nature. These art-recognized materials may include Kraft, wood pulp and mechanical wood pulp. As used herein, the term "mechanical wood pulp" is meant to include ground wood pulp, thermomechanical pulp and refiner wood pulp. The common characteristic of these mechanical pulps is that no attempt has been made to separate the fibers by chemical means although they may later, after being reduced to fine particulate matter, be subjected to chemical treatment. Preferably, when mechanical wood pulp is used in this invention, such mechanical wood pulp has a Canadian Standard Freeness (TAPPI TEST METHOD T-227) of from about 60-500 and preferably from about 150-300. Another valuable material used in combination with peat moss is Kraft wood pulp. This material is generally a chemically treated, long fibered pulp such as sulfite and sulfate wood pulps. A suitable mixture of ingredients for the absorbent materials of the invention may comprise from about 5 to about 20 percent by weight of Kraft wood pulp, with the remainder being essentially peat moss. It is understood that these compositions are preferred peat moss embodiments and that those familiar with the art may find a wide range of peat moss compositions as well as other absorbent materials, i.e., superabsorbents, for use with the products of this invention.

The above peat mixtures can be additionally combined with fibrous means comprising polymer fibers, preferably polyester fibers, and most preferably staple-length polyester fibers. The preferred polyester fibers are disposed to maintain the integrity of the product during processing and use, without subtracting from the intended mechanical flexibility. Preferably the polyester material or other suitable fibrous material is slurried with water in a pulper prior to being mixed with the preferred peat moss compositions. The preferred absorbent members 29 (exclusive of tissue wrap 22) of this invention can comprise about 2.5% to about 20.0% polyester by weight, preferably about 4.0% to 8.0% polyester by weight. The term "peat moss", then, as used herein is intended to include peat moss products comprising, in addition to particles of peat moss, cellulosic fibers and polymer fibers as described above.

The mixture of screened peat moss and selected fibers are processed into an absorbent member by methods known in the art. Generally, the screened peat moss and selected fibers are slurried together to form an aqueous slurry which is preferably flowed onto a Fourdrinier wire and dewatered to form the starting material for absorbent member 29. In the most preferred embodiment, a laminate is then made from the peat board and layers of Kraft wood pulp. The resulting composite (numeral 21 in the Figures) is then calendared prior to use as a structural component of device 10.

Absorbent members prepared as described above tend not to be flexible enough for use in the present invention without undergoing "tenderizing", i.e., processing by any number of known methods to increase the flexibility of the board. See, for example, the disclosure of U.S.S.N. 242,274, filed September 12, 1988, * which discloses a method of tenderizing the absorbent member by a special cutting process which severs the peat moss portions of the member but which leaves those portions linked with fibrous means to produce a hinge-like effect in the resulting member. Other "tenderizing" methods which might be employed to enhance flexibility are microcorrugation and perfembossing as disclosed in U.S. Patent No. 4,605,402 to Iskra.
* see EP-A-0360472

One or more layers of peat moss sheet may be used as the absorbent member or, as shown in the preferred embodiments in the Figures, a single sheet may be used and folded back upon itself in a C-configuration. Obviously, the greater the absorptive capacity which is desired for the final device, the more absorptive capacity must be provided in the absorbent member. As peat moss sheets decrease in flexibility with thickness, it may be desired, as illustrated, to utilize a single thin sheet, folded back on itself, or two thin sheets placed on atop the other, in lieu of one thicker, less flexible sheet. Excellent results have been attained utilizing a peat moss sheet having a basis weight of 400 g/m². For a regular urinary pad, it may be desirable to use a peat moss absorptive element weighing approximately 15.5 g, and for a urinary device with greater absorptive capabilities, it may be desirable to use a peat moss absorptive member weighing approximately 21.2 g.

Other highly absorbent materials which can be utilized as the absorbent member in the device of this invention are densified sheets of materials comprising blends of wood pulp and superabsorbent materials. Such blends may contain, in addition, synthetic pulp (Pulpex®) or synthetic fibers such as polyolefin fibers as binder materials.

The superabsorbents incorporated into or blended with the pulp to form the above-described absorbent member are well known materials and are generally water-insoluble, water-swellable polymeric substances capable of absorbing water in an amount which is at least 10 times the weight of the substance in their dry form. The superabsorbent is in the form of powders, fibers, spheres, particles, bits of film, globules, or the like. The superabsorbent prepared by polymerization of a monomer solution placed on fibers in a web is most frequently in the form of globules and bits of film-like particles in the web structure.

One type of superabsorbent material provides particles or fibers which may be described chemically as having a backbone of natural or synthetic polymers with hydrophilic groups or polymers containing hydrophilic groups being chemically bonded to the backbone or an intimate mixture therewith. Included in this class of materials are such modified natural and regenerated polymers as polysaccharides including, for example, cellulose and starch and regenerated cellulose which are modified by being carboxylalkylated, phosphonoalkylated, sulfoalkylated, or phosphorylated to render them highly hydrophilic. Such modified polymers may also be crosslinked to improve their water-insolubility.

These same polysaccharides may also serve, for example, as the backbone on to which other polymer moieties may be bonded by graft copolymerization techniques. Such grafted polysaccharides and their method of manufacture are described in U.S. Patent No. 4,105,033 to Chatterjee et al.

In addition to the modified natural and regenerated polymers, the hydrocolloid component may comprise wholly synthetic hydrophilic particles. Examples of those now known in the art are polyacrylonitrile fibers which may be modified by grafting moieties thereon such as polyvinylalcohol chains, polyvinyl alcohol itself, hydrophilic polyurethane, poly(alkyl phosphonates), partially hydrolyzed polyacrylamides (e.g., poly (N-N-dimethylacrylamide), sulfonated polystyrene, or a class of poly(alkyleneoxide). These highly hydrophilic synthetic polymers may be modified by other chemical treatments such as crosslinking or hydrolysis. Further examples known in the art are the non-ionic polymers such as polyoxyethylene, polyoxypropylene, and mixtures thereof which have been suitably cross linked, either chemically or by irradiation. Still another more recent type is a derivative of isobutylene-maleic anhydride copolymer.

The material from which the receiving layer is made generally contains wood pulp fibers. Wood pulp is highly wettable, but collapses when wet. Non-cellulosic synthetic fibers such as but not limited to polyolefin fibers (polyethylene, polypropylene, and bicomponent fibers) resist wet collapse due to their hydrophobicity. Thus, by blending these two materials together, a receiving layer with an optimum absorbency can be obtained. Blending about 5 to 50 weight %, preferably about 15 - 30 weight %, of non-cellulosic synthetic fibers with the wood pulp fibers, in accordance with the invention, leads to a material with markedly improved wet collapse properties compared to 100% wood pulp, but which substantially retains the wettability properties of the wood pulp. As mentioned earlier, in the preferred embodiment of this invention, the receiving layer comprises approximately 80% by weight wood pulp and approximately 20% bicomponent fiber (polyester core/polyethylene sheath fibre of 3d and 3.2 cm (1¼") length) and weighs 271 g/m² (8 oz/yd²).

A receiving layer of wood pulp/non-cellulosic synthetic fiber can be prepared by methods known in the art utilizing a transverse webber. A sheet of a combined pulp/synthetic fiber material may be fed to the webber, processed so as to individualize the fibers and then fed into an air stream. The processed pulp/synthetic fiber is collected, and thermally bonded to produce a stable web. Such method is disclosed in U.S. Patent Application Serial No. 99,875, filed September 22, 1987.**
**see EP-A-0307967

Both the receiving layer 23 and the absorbent member 29 may be substantially rectangular in shape, reducing wasteful cut-outs. Typical dimensions in an adult urinary device for these elements would be a 12.7 cm x 35.6 cm (5" x 14") receiving layer 23 covering a 6.4 cm x 30.5 cm (2.5" x 12") absorbent member 29.

The liquid-permeable facing 14 provided on the urinary device 10 of the present invention may be an apertured film, a nonwoven fabric, or a similar material having a high degree of moisture permeability. For example, the nonwoven fabric may comprise polyester, polyethylene, polypropylene, bicomponent, nylon, rayon, or the like fibers. Preferably, the nonwoven fabric used for the cover has a basis weight in the range of 10.2 to 170 g/m² (0.3 to 5.0 oz. per square yard) and has a density less than 0.2 gms/cc. The most suitable nonwoven fabrics have high loft, softness and drape characteristics.

It will be understood that numerous variations and modifications may be effected without departing from the scope of the present invention.

The standard test used to measure stiffness is as follows:

### Stiffness Test

A Gurley Stiffness Tester (W. and L.E. Gurley, Troy, New Jersey), an instrument using a cantilever principle to measure the force required to move a specimen a certain distance, is utilized. The sample is mounted in a clamp, and a small synchronous motor rotates the clamp and the sample in a horizontal direction. The sample presses against the bottom of a pointer, the top of which extends to a calibrated dial. The maximum displacement of the dial by the sample is read and converted to stiffness.

Samples of the material to be tested are cut, the samples being (5.1 cm x 3.8 cm (2" x 1½") in dimension and, in the case of measuring MD stiffness, having the 3.8 cm (1½") dimension in the machine direction. The testing instrument is checked to see that it is level. There are various weights to be inserted into the vane of the tester (5 gms, 25 gms, 50 gms, 200 gms) at 10.2 cm (4"), 5.1 cm (2") and 2.5 cm (1") locations. The appropriate weight and location are selected to give a reading between 2 and 8 on the scale. This can only be achieved by following the remainder of this procedure and testing some trial samples.

The 5.1 cm (2") side of the sample is clamped centrally in the sample holder, being sure that the sample is seated all the way up so that the bottom of the sample is parallel to the vane. The instrument is then operated so as to drive the arm carrying the clamped sample into contact with the pointer. This action causes the pointer to move indicating a reading on the scale. The maximum reading, obtained as the sample clears the pointer, is noted. The instrument is then operated to move the sample in the opposite direction and again the highest scale reading is noted. These two readings are averaged, and the result recorded. Readings are taken for three samples and averaged. After average values have been calculated, reference is made to the conversion table on the instrument to determine the factor to be used for converting scale readings to milligrams stiffness. The factor is determined by the sample size, the weight value in grams that was inserted into the vane, and the distance of the weight from the center of the vane in inches. A final reading is obtained by multiplying the average reading by this factor.

## Claims

1. A generally U-shaped absorbent product having a central crotch portion (40) and adjoining end portions (41, 42), said central crotch portion having upstanding side walls (36a, 36b) defining the height of said absorbent product in said crotch portion and a bottom wall defining the width of said product in said crotch portion, said absorbent product comprising
a liquid-impermeable backing sheet (12);
an absorbent unit (13) comprising first (29) and second (23) portions exhibiting different degrees of stiffness, said first portion comprising a first discrete layer (21) of absorbent material and said second portion comprising a second discrete layer (23) of absorbent material, said first portion being the stiffer portion and being positioned along the bottom wall of said crotch portion, and said second portion being the less rigid portion and extending at least partially up the side walls of said crotch portion; and
a liquid permeable facing (14) adhered to said backing sheet so as to entrap said absorbent unit therebetween; and elastic means (15) along the side edges of said product in said crotch portion,
characterized in that said second portion (23) of said absorbent unit has an absorptive capacity which is less than the absorptive capacity of said first portion (29), said absorptive capacity of said second portion being at least about 12 g water per gram of said second portion, and said second portion (23) including a web comprising about 50 to 95 weight percent wood pulp and about 5 to 50 weight percent synthetic fibers.

2. The absorbent product of claim 1 which comprises a disposable urinary pad.

3. The absorbent product of claim 1 or claim 2 wherein said elastic means (15) have an overall contraction force of about 40 to 140 grams.

4. The absorbent product of any preceding claim wherein the MD stiffness of said first portion (29) of said absorbent unit (13) is in the range of about 2000 to 8000 mg.

5. The absorbent product of any preceding claim wherein the MD stiffness of said second portion of said absorbent unit (13) is in the range of about 200 to 3000 mg.

6. The absorbent product of any preceding claim wherein the height of said product in said crotch portion (40) is at least about 2.54 cm (about one inch).

7. The absorbent product of any preceding claim wherein the angle at which said side walls (36a, 36b) join said bottom wall of said crotch portion (40) is within the range of about 60 to 120°.

8. The absorbent product of any preceding claim wherein said second portion (23) is a web comprising about 70-85 weight percent wood pulp and about 15 to 30 weight percent synthetic fibers.

9. The absorbent product of any preceding claim wherein said synthetic fibers are bicomponent fibers.

10. The absorbent product of claim 9 wherein said bicomponent fibers comprise a core of polyester and a sheath of polyethylene.

11. The absorbent product of any preceding claim wherein said first portion (29) comprises at least one layer of peat moss sheet.

12. The absorbent product of any preceding claim wherein said second portion (23) is disposed upwardly so as to face the wearer of the absorbent product, and is larger in dimension than said first portion (29) so as to substantially cover said first portion.

13. The absorbent product of any preceding claim wherein said elastic means (15) comprise rubber threads adhered intermittently to said pad.

14. The absorbent product of any preceding claim wherein said backing sheet (12) is a polyolefin film.

15. The absorbent product of claim 14 wherein said backing sheet (12) is a polyethylene film.

16. The absorbent product of any preceding claim wherein adhesive means (16) are positioned on the bottom surface (12b) of said backing sheet (12) for temporarily, but securely, adhering said backing to the crotch portion of the wearer's garment.

17. The absorbent product of any preceding claim wherein said liquid permeable facing (14) is selected from films and fabrics of polyester, polyethylene, bicomponent fibers, nylon and rayon.

18. The absorbent product of claim 17 wherein said liquid permeable facing (14) comprises a thermally bonded web of polyester/polyethylene bicomponent fiber.

## Patentansprüche

1. Allgemein U-förmiger saugfähiger Gegenstand mit einem mittleren Schrittbereich (40) und daran anschließenden Endbereichen (41, 42), wobei der mittlere Schrittbereich hochstehende Seitenwände (36a, 36b), welche die Höhe des saugfähigen Gegenstandes im Schrittbereich festlegen, und eine die Breite des Gegenstandes im Schrittbereich festlegende Bodenwand umfaßt, wobei der saugfähige Gegenstand umfaßt:
eine flüssigkeitsundurchlässige Rückschicht (12);
eine saugfähige Einheit (13) mit ersten (29) und zweiten (23) Bereichen, die verschiedene Steifigkeitsgrade aufweisen, wobei der erste Bereich eine erste diskrete Schicht (21) eines saugfähigen Materials und der zweite Bereich eine zweite diskrete Schicht (23) eines saugfähigen Materials aufweist, und der erste Bereich der steifere Bereich ist und entlang der Bodenwand des Schrittbereichs angeordnet ist und der zweite Bereich der weniger steife Bereich ist und an den Seitenwänden des Schrittbereichs zumindest teilweise hochsteht; und
eine flüssigkeitsdurchlässige Vorderschicht (14), die an der Rückschicht anhaftet, um zwischen diesen die saugfähige Einheit einzuschließen; und elastische Mittel (15) entlang der Seitenränder des Gegenstandes im Schrittbereich,
dadurch gekennzeichnet, daß der zweite Bereich (23) der saugfähigen Einheit eine Saugfähigkeit aufweist, die geringer ist als die Saugfähigkeit des ersten Bereichs (29), wobei die Saugfähigkeit des zweiten Bereichs mindestens etwa 12 g Wasser pro Gramm Material des zweiten Bereichs beträgt und der zweite Bereich (23) eine Matte umfaßt, die ca. 50 bis 95 Gewichtsprozent Zellstoff und ca. 5 bis 50 Gewichtsprozent synthetische Fasern enthält.

2. Saugfähiger Gegenstand nach Anspruch 1, der ein Wegwerf-Urinkissen umfaßt.

3. Saugfähiger Gegenstand nach Anspruch 1 oder 2, wobei die elastischen Mittel (15) eine Gesamtkontraktionskraft von ca. 40 bis 140 g aufweisen.

4. Saugfähiger Gegenstand nach einem der vorhergehenden Ansprüche, wobei die erforderliche Maximalsteifigkeit des ersten Bereichs (29) der saugfähigen Einheit (13) im Bereich von ca. 2000 bis 8000 mg liegt.

5. Saugfähiger Gegenstand nach einem der vorhergehenden Ansprüche, wobei die erforderliche Maximalsteifigkeit des zweiten Bereichs der saugfähigen Einheit (13) im Bereich von ca. 200 bis 3000 mg liegt.

6. Saugfähiger Gegenstand nach einem der vorhergehenden Ansprüche, wobei die Höhe des Gegenstandes im Schrittbereich (40) mindestens ca. 2,54 cm (ca. ein Zoll) beträgt.

7. Saugfähiger Gegenstand nach einem der vorhergehenden Ansprüche, wobei dery Winkel, unter dem die Seitenwände (36a, 36b) auf die Bodenwand des Schrittbereichs (40) treffen, im Bereich von ca. 60 bis 120° liegt.

8. Saugfähiger Gegenstand nach einem der vorhergehenden Ansprüche, wobei der zweite Bereich (23) eine Matte ist, die ca. 70 bis 85 Gewichtsprozent Zellstoff und ca. 14 bis 30 Gewichtsprozent synthetische Fasern enthält.

9. Saugfähiger Gegenstand nach einem der vorhergehenden Ansprüche, wobei die synthetischen Fasern Zweikomponenten-Fasern sind.

10. Saugfähiger Gegenstand nach Anspruch 9, wobei die Zweikomponenten-Fasern einen Kern aus Polyester und einen Mantel aus Polyethylen aufweisen.

11. Saugfähiger Gegenstand nach einem der vorhergehenden Ansprüche, wobei der erste Bereich (29) mindestens eine Lage aus einer Torfmoosschicht umfaßt.

12. Saugfähiger Gegenstand nach einem der vorhergehenden Ansprüche, wobei der zweite Bereich (23) nach oben gerichtet und dem Träger des saugfähigen Produktes zugewandt ist und in den Maßen größer ist als der erste Bereich (29), um auf diese Weise den ersten Bereich im wesentlichen zu bedecken.

13. Saugfähiger Gegenstand nach einem der vorhergehenden Ansprüche, wobei die elastischen Mittel (15) Gummifäden umfassen, die intermittierend am Kissen angebracht sind.

14. Saugfähiger Gegenstand nach einem der vorhergehenden Ansprüche, wobei die Rückschicht (12) ein Polyolefin-Film ist.

15. Saugfähiger Gegenstand nach Anspruch 14, wobei die Rückschicht (12) ein Polyethylen-Film ist.

16. Saugfähiger Gegenstand nach einem der vorhergehenden Ansprüche, wobei Haftmittel (16) an der Bodenfläche (12b) der Rückschicht (12) vorgesehen sind, yum zeitweilig aber gesichert die Rückschicht an dem Schrittbereich der Wäsche des Trägers zu befestigen.

17. Saugfähiger Gegenstand nach einem der vorhergehenden Ansprüche, wobei die flüssigkeitsdurchlässige Vorderschicht (14) aus Filmen und Geweben aus Polyester, Polyethylen, Zweikomponenten-Fasern, Nylon und Rayon ausgewählt ist.

18. Saugfähiger Gegenstand nach Anspruch 17, wobei die flüssigkeitsdurchlässige Vorderschicht (14) eine thermisch verbundene Matte aus Polyester/Polyethylen Zweikomponenten-Fasern umfaßt.

## Revendications

1. Produit absorbant de forme globale en U, comprenant une partie centrale d'entrecuisse (40) et des parties contiguës d'extrémité (41, 42), ladite partie centrale d'entrecuisse comprenant des parois latérales orientées vers le haut (36a, 36b) qui déterminent la hauteur dudit produit absorbant dans ladite partie d'entrecuisse, ainsi qu'une paroi de fond déterminant la largeur dudit produit dans ladite partie d'entrecuisse, ledit produit absorbant comprenant
une feuille de soutien (12) imperméable aux liquides ;
un assemblage absorbant (13) comprenant des première (29) et seconde (23) parties ayant des degrés différents de rigidité, ladite première partie consistant en une première couche individuelle (21) de matière absorbante et ladite seconde partie consistant en une seconde couche individuelle (23) de matière absorbante, ladite première partie étant la partie la plus rigide et étant placée le long de la paroi de fond de ladite partie d'entrecuisse et ladite seconde partie étant la partie la moins rigide et se prolongeant au moins partiellement vers le haut sur les parois latérales de ladite partie d'entrecuisse ; et
un parement perméable aux liquides (14) collé à ladite feuille de soutien de façon à emprisonner l'assemblage absorbant entre eux ; et un moyen élastique (15) situé le long des bords latéraux dudit produit dans ladite partie d'entrecuisse,
caractérisé en ce que ladite seconde partie (23) dudit assemblage absorbant a une capacité d'absorption qui est inférieure à la capacité d'absorption de ladite première partie (29), ladite capacité d'absorption de ladite seconde partie étant au moins d'environ 12 g d'eau par gramme de ladite seconde partie et ladite seconde partie (23) se composant d'une étoffe constituée d'environ 50 à 95 pour cent en poids de pulpe de bois et d'environ 5 à 50 pour cent en poids de fibres synthétiques.

2. Produit absorbant selon la revendication 1, qui comprend un tampon urinaire jetable.

3. Produit absorbant selon la revendication 1 ou la revendication 2, dans lequel ledit moyen élastique (15) a une force globale de contraction d'environ 40 à 140 grammes.

4. Produit absorbant selon l'une quelconque des revendications précédentes, dans lequel la rigidité sous charge moyenne de ladite première partie (29) dudit assemblage absorbant (13) est de l'ordre d'environ 2000 à 8000 mg.

5. Produit absorbant selon l'une quelconque des revendications précédentes, dans lequel la rigidité sous charge moyenne de ladite seconde partie dudit assemblage absorbant (13) est de l'ordre d'environ 200 à 3000 mg.

6. Produit absorbant selon l'une quelconque des revendications précédentes, dans lequel la hauteur dudit produit dans ladite partie d'entrecuisse (40) est au moins d'environ 2,54 cm (environ 1 pouce).

7. Produit absorbant selon l'une quelconque des revendications précédentes, dans lequel l'angle suivant lequel lesdites parois latérales (36a, 36b) rejoignent ladite paroi de fond de ladite partie d'entrecuisse (40) est de l'ordre d'environ 60 à 120°.

8. Produit absorbant selon l'une quelconque des revendications précédentes, dans lequel ladite seconde partie (23) est une étoffe se composant d'environ 70-85 pour cent en poids de pulpe de bois et d'environ 14 à 30 pour cent en poids de fibres synthétiques.

9. Produit absorbant selon l'une quelconque des revendications précédentes, dans lequel lesdites fibres synthétiques sont des fibres à deux composants.

10. Produit absorbant selon la revendication 9, dans lequel lesdites fibres à deux composants comprennent une âme de polyester et une gaine de polyéthylène.

11. Produit absorbant selon l'une quelconque des revendications précédentes, dans lequel ladite première partie (29) comprend au moins une couche formée d'une feuille de tourbe.

12. Produit absorbant selon l'une quelconque des revendications précédentes, dans lequel ladite seconde partie (23) est orientée vers le haut de manière à se trouver en face de l'utilisateur du produit absorbant et a une dimension plus grande que celle de ladite première partie (29) de manière à recouvrir sensiblement ladite première partie.

13. Produit absorbant selon l'une quelconque des revendications précédentes, dans lequel ledit moyen élastique (15) consiste en des fils de caoutchouc collés par intermittences audit tampon.

14. Produit absorbant selon l'une quelconque des revendications précédentes, dans lequel ladite feuille de soutien (12) est un film de polyoléfine.

15. Produit absorbant selon la revendication 14, dans lequel ladite feuille de soutien (12) est un film de polyéthylène.

16. Produit absorbant selon l'une quelconque des revendications précédentes, dans lequel lesdits moyens adhésifs (16) sont placés sur la surface du fond (12b) de ladite feuille de soutien (12) pour coller temporairement, mais étroitement ledit soutien à la partie d'entrecuisse du vêtement de l'utilisateur.

17. Produit absorbant selon l'une quelconque des revendications précédentes, dans lequel ledit parement (14) perméable aux liquides est choisi parmi des films et des étoffes de polyester, polyéthylène, fibres à deux composants, nylon et rayonne.

18. Produit absorbant selon la revendication 17, dans lequel ledit parement perméable aux liquides (14) consiste en une étoffe liée à la chaleur et formée de fibres à deux composants de polyester et de polyéthylène.
